# EUROPEAN PATENT SPECIFICATION

(11) **EP 0 414 407 B1**
(45) Date of publication and mention of the grant of the patent: **15.12.1993**
(21) Application number: 90308659.3
(22) Date of filing: 07.08.1990
(51) Int. Cl.: C07C 233/18, C07C 233/69, C08K 5/20

(54) **N-acyl and O-acyl derivatives of N,N-bis(2,2-dimethyl-3-hydroxypropyl)amine, their preparation and use**
N-Acyl- und O-Acyl-Derivate von N,N-bis(2,2-Dimethyl-3-Hydroxypropyl)amin, ihre Herstellung und Verwendung
Dérivés N-acyl et O-acyl de la N,N-bis(2,2-diméthyl-3-hydroxypropyl)amine, leur préparation et utilisation

(30) Priority: 21.08.1989 FI 893914
(43) Date of publication of application: 27.02.1991
(73) Proprietor: NESTE OY, FIN-02150 Espoo (FI)
(72) Inventor: Lahtinen, Leila, SF-06850 Kulloo (FI); Tuominen, Simo, SF-06850 Kulloo (FI); Koskimies, Salme, SF-00930 Helsinki (FI); Valtonen, Eija, SF-06400 Porvoo (FI)
(74) Representative: Lamb, John Baxter

(56) References cited:
- EP-A- 0 046 288
- FI-A- 83 212
- FR-A- 1 494 701
- US-A- 3 179 615
- US-A- 3 236 403

## Description

The present invention relates to N-acyl and O-acyl derivatives of N,N-bis(2,2-dimethyl-3-hydroxypropyl)- amine, their production and their use.

The new compounds of the invention may be represented by the formula: 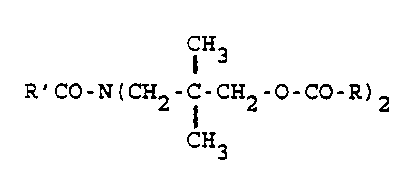
where R and R' are the same as or different and each is a C₁-C₁₀ alkyl or C₆-C₁₀ aryl group.

N-acyl, O-acyl derivatives of 2-hydroxyethylamine are disclosed in US-A-3179615 and N-acyl, O-acyl derivatives of possibly substituted 2-hydroxyethylamine are disclosed in FR-A-1494701. It is also known in the art that primary and secondary amines react with formaldehyde and isobutyrlaldehyde (EP 46288, and Arch. Pharmaz. 308/75 p.352), generating either straight chain or cyclic Mannich reaction products (equations 1 and 2).

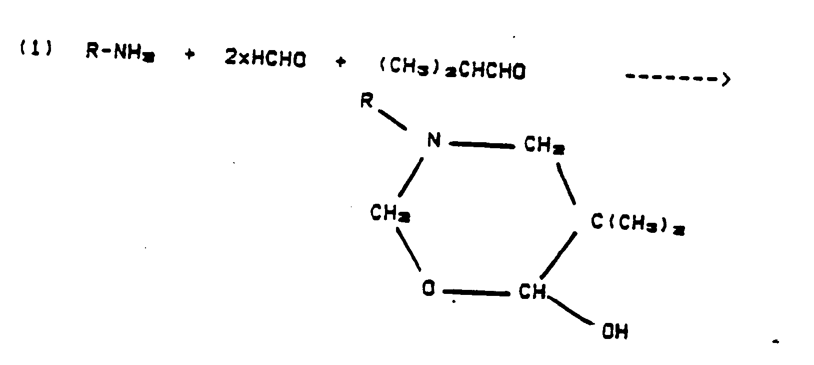

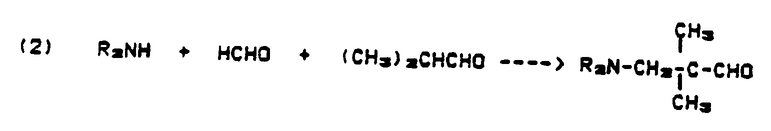

In the present invention a modified Mannich reaction is used as an intermediate step when various N-acyl derivatives of N,N-bis(2,2-dimethyl-3-hydroxypropyl)amine are prepared according to the following reaction (equations: 3-5).

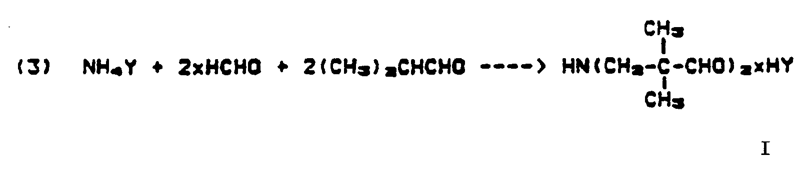

This condensation reaction (3) (in which the group Y can be a halide or sulphate group) can be performed at 50 to 90°C in about three hours, either under reflux or under slight overpressure, suitably using paraformaldehyde, trioxane or an aqueous 40% formaldehyde solution for the formaldehyde source.

The resultant compound is thereafter reduced according to the following reaction equation (4)

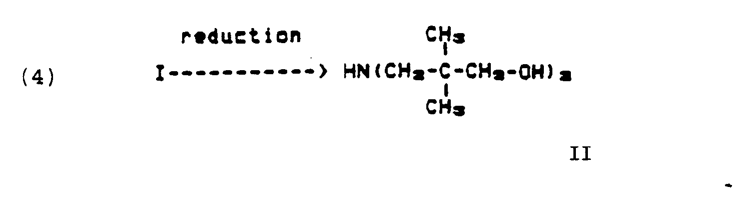

The reduction reaction (4) may be performed using several conventional reduction procedures, e. g. by hydrogen reduction in the presence of Ni, Pt, Pd, or of a transition metal catalyst, or by hydride reduction (NaBH4, LiAiH4, etc. ). Particularly suitable are, eg., reduction with NaBH4 at 15-20°C, or with hydrogen in the presence of a Raney-Ni catalyst at 70-75°C at 4.05-5.07 atm pressure over about four hours.

Thereafter, the reduced product is acylated (equation 5).

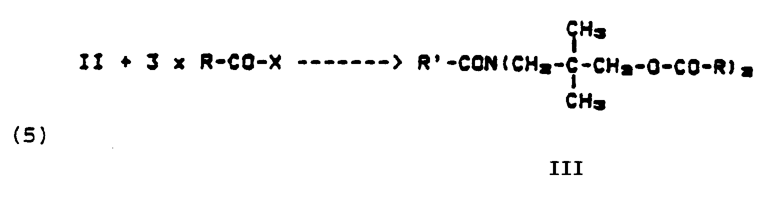

In equation 5, R and R′ are the same or different alkyl or aryl groups which may contain up to 10 carbon atoms. X refers to a halide or an acyloxy group.

Esterification and N-acylation of the compound II can be performed simultaneously using a suitable carboxylic acid, carboxylic acid chloride oranhydride. For acylation also e.g. a mixture of acid chlorides may be used. It is particularly suitable to use acid chloride at 20-100°C when the reaction time is 2 to 20 hrs.

The invention thus relates to the production and use of compounds of type III. Possible applications of type III compounds are stabilization of organic compounds against oxidation and against the effect of heat or radiation, and against colour formation. Stabilzation of polymers, in particular of PVC and polyolefins (homopolymers and copolymers) use of PVC as plasticizer, use as lubricating agent, emulsifier, corrosion resistance agent, and as metal complexing agents.

The compounds of the invention of type III are novel in their chemical formula. Their production, identification and testing, particularly for PVC plasticizer and stabilation use, is described more in detail in the following examples.

### Example 1

### Item A:

In a reactor provided with a mixer, a reflux condenser, and thermometer, 66.9 g ammonium chloride, 162 g formaline and 144 g isobutyraldehyde, and 1 ml hydrochloric acid were charged. The mixture was heated to 60°C, whereby the isobutyraldehyde started to be refluxed. Towards the end of the refluxation the temperature was raised to 80°C at which the boiling was continued for 2.5 hours. The obtained condensation product (362 g) was added at 18 to 25°C to a reactor provided with mechanical stirring, in which 209 g of 12% NaBH4 solution in aqueous 40% NaOH and 500 ml methanol were added. After adding the condensation product, stirring was continued for one more hour, whereafter the water and the methanol were distilled off. The produced boric compounds were dispersed with 1000 ml water by heating in a water bath. The organic phase was separated when hot, and the product was isolated by vacuum distillation. The product (II) was identified with IH-NMR spectrophotometer and the GC-MS. The melting point of the product is 28-30°C, and the yield was 62%.

### Item B:

9.5 g of amino alcohol (II) were dissolved in a mixture of dry ether (30 ml) and pyridine (13.0 g). 25.7 g of octanoyl chloride dissolved in ether (10 ml) was added at room temperature in about five minutes whereafter the reaction mixture was refluxed further for three hours. The reaction mixture was washed with diluted hydrochloric acid, mild sodium hydroxide solution and, finally, with water to become neutral. The yield of the raw product obtained after the drying and evaporation of the dissolvent was 20 g and the purity of the amidodester was 93%. The product was identified with the GC-MS system.

### Example 2

### Item A:

The pH of the condensation product (362 g) obtained as in item A in Example 1 was set to value 5 by a mild (5%) NaOH solution. The product, 15.0 g of Raney nickel and 100 ml water were added in a 1 liter autoclave provided with a bottom tube. The air was removed from the autoclave by nitrogen-hydrogen rinses. The temperature of the autoclave was raised to 65°C and the hydrogen pressure to (4.05 MPa to 40 atm.) The pressure was maintained almost constant throughout the entire reaction (5 hrs) by periodic hydrogen supply.

After being hydrated, the catalyst was centrifuged off and the product was neutralized with 25% NaOH solution. The product released from the salt mode was extracted into diethyl ether, and the organic phase was dried with sodium sulphate. The raw product obtained after the evaporation of ether was purified by vacuum distillation (125-150°C, 1-3 . 10⁻³ atm). Identification as above. The yield was 54%.

### Item B:

9.5 g of the amino alcohol (II) obtained in A were dissolved in a mixture of dry ether (50 ml) and pyridine (13.0 g). 25.7 g of 2-ethyl hexanoyl choride, dissolved in ether (30 ml) were added at room temperature in about five minutes, whereafter the reaction mixture was refluxed for 7 hours at the boiling point of ether and was thereafter stirred for 20 hours at room temperature. The reaction mixture was washed and treated as in Example I/B. The yield of the raw product was 19 g and the purity of the amidodiester was 94%. The product (III, in which R is 1-ethyl pentyl) was identified by the GC MS system.

### Example 3

The reaction described in B of Example 1 was repeated using 9.5 g of amino alcohol II and 25.7 g n-octanoyl chloride, but instead of pyridine, 16.7 g triethyl amine were used. The reaction time was 20 hours. The yield of the raw product was 22 g and purity 72.5%.

### Example 4

The reaction described in B of Example 1 was repeated with 9.5 g of amino alcohol (II), but instead of the octanoyl chloride, 22.2 g benzoyl chloride were used. The reaction time was 20 hours. The reaction mixture was aftertreated as in Example 1/C. The yield of the yellow oily product was 45%. (Identification GC-MS).

### Example 5

The reaction described in B of Example 1 was repeated, but instead of the octanoyl chloride, 13 g acetyl chloride were used. The yield of the raw product was 20% and purity 60%.

### Example 6

15.6 g amino alcohol, 41.9 g pyridine, and 27 g acetic anhydride were charged in a 250 ml glass reactor provided with a reflux condenser. The reaction mixture was mixed at 120°C for about three hours. During the reaction, nitrogen protection gas blanket was used. In the cooled mixture, 40 ml water and 100 ml ether were added. The organic phase was separated and it was washed with dilute hydrochloric acid, sodium hydrogen carbonate, and finally, to make it neutral, with water. The yield of the acetamido derivative III was 47%. The product was identified by the GC-MS; m/e 315 (M+1).

Compounds having the structural formula III were tested in PVC applications. For instance, a N-octanoyl derivative of dioctanoyl ester of aminodialcohol (R = R' = n heptyl) serves as PVC plasticizer comparable to dioctylphthalate. In addition, the compound A possesses an effect increasing the thermal stability of PVC when used together with Cd/Zn stabilizer. This can be seen in the VDE value representing thermal stability (the thermal stability values are determined according to the VDE standard (3.69)).

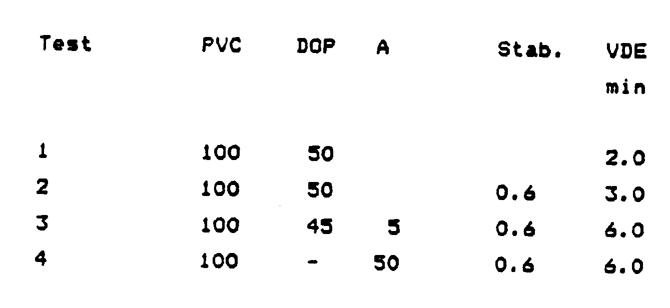

## Claims

1. N-acyl and O-acyl derivatives of N,N-bis(2,2-dimethyl-3-hydroxypropyl)amine.

2. Compounds according to claim 1 having the formula: 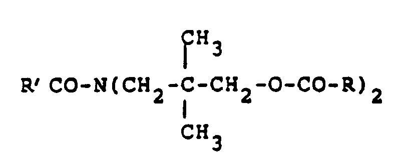 where R and R' are the same or are different and each is a C₁-C₁₀ alkyl or C₆-C₁ aryl group.

3. A process for the production of compounds according to claim 1 or claim 2, characterized in that an ammonium halide or sulphate, formaldehyde and isobutyraldehyde are reacted together according to equation 3. 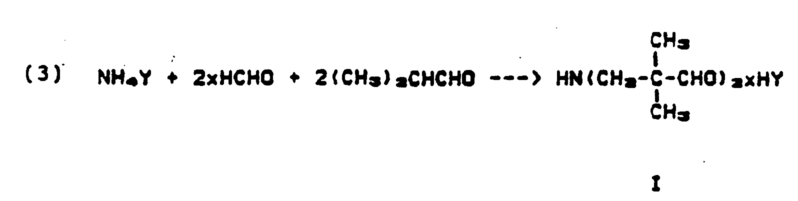 (where Y is halide or sulphate) the condensation product from which is then reduced according to equation 4. 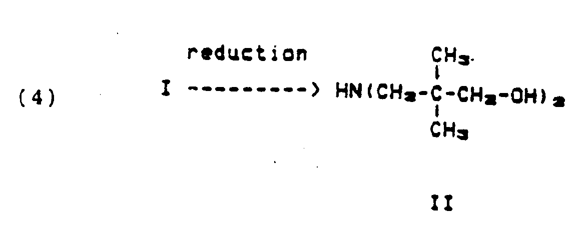 whereafter the reduced product is acylated according to equation 5. 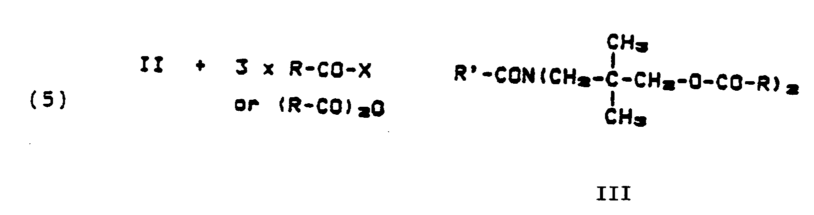 where R and R' are the same or different alkyl groups which may contain up to 10 carbon atoms; and X is a halide or acyloxy group.

4. A process according to claim 3, characterized in that in the condensation reaction of equation 3, paraformaldehyde, trioxane, or an aqueous 40% formaldehyde solution is used as formaldehyde source.

5. A process according to claim 3 or claim 4, characterized in that the condensation reaction according to equation 3 is performed at 50-90°C under reflux or under a small overpressure.

6. A process according to any one of claims 3-5, characterized in that the reduction reaction 4 is performed at 15-20°C by hydrogen reduction in the presence of Ni, Pt, Pd or a transition metal catalyst, or by hydride reduction, or with hydrogen in the presence of the Raney-Ni catalyst at 70-75°C at 40-55 atm pressure.

7. A process according to any one of claims 3-6, characterized in that the esterification reaction 5 is performed and the N-acylation is performed using an appropriate carboxylic acid, carboxylic acid chloride or anhydride at 20-100°C.

8. The use of compounds according to claim 1 for plasticizers and stabilizing agents in polymers (in particular in PVC polymer) as lubricating agents, emulsifiers, corrosion resistance agent, and as metal complexing agents.

## Patentansprüche

1. N-Acyl- und O-Acylderivate des N,N-bis (2,2-dimethyl-3-hydroxypropyl)amins.

2. Verbindungen gemäß Anspruch 1 mit der Formel: 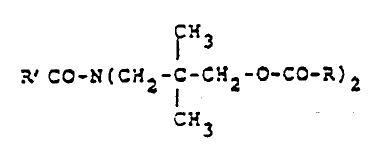 wobei R und R' gleich oder verschieden sind und jeweils eine C₁-C₁₀-Alkyl- oder C₆-C₁₀-Arylgruppe bedeuten.

3. Verfahren zur Herstellung der Verbindungen gemäß Anspruch 1 oder Anspruch 2, dadurch gekennzeichnet daß ein Ammoniumhalogenid oder Ammoniumsulfat, Formaldehyd und Isobutyraldehyd gemäß Gleichung 3 miteinander umgesetzt werden, 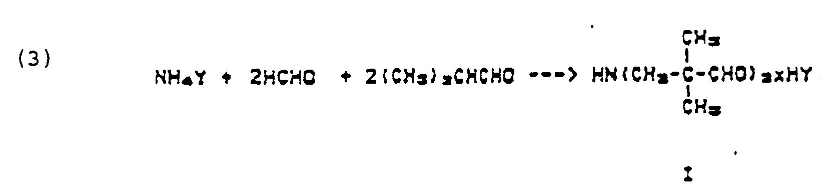 (wobei Y Halogenid oder Sulfat bedeutet), deren Kondensationsprodukt dann gemäß Gleichung 4 reduziert wird, 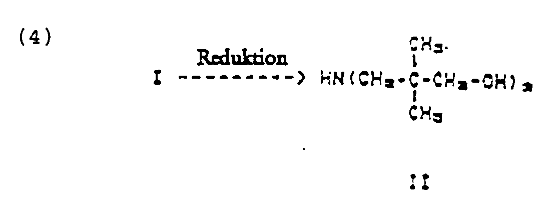 worauf das reduzierte Produkt gemäß Gleichung 5 acyliert wird, 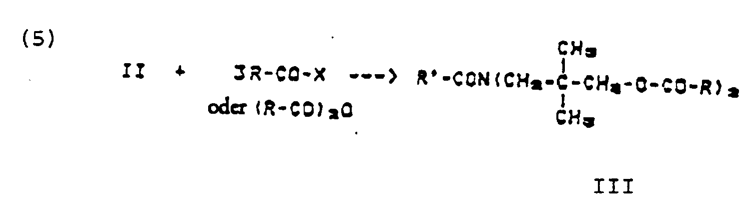 wobei R und R' gleiche oder verschiedene Alkylgruppen bedeuten, die bis zu 10 Kohlenstoffatomen enthalten können, und X eine Halogenid- oder Acyloxygruppe bedeutet.

4. Verfahren gemäß Anspruch 3, dadurch gekennzeichnet, daß bei der Kondensationsreaktion der Gleichung 3 Paraformaldehyd, Trioxan oder eine wäßrige 40%-ige Formaldehydlösung als Formaldehydquelle eingesetzt wird.

5. Verfahren gemäß Anspruch 3 oder 4, dadurch gekennzeichnet, daß die Kondensationsreaktion gemäß Gleichung 3 bei 50 bis 90°C unter Rückfluß oder unter leichtem Überdruck durchgeführt wird.

6. Verfahren gemäß einem der Ansprüche 3-5, dadurch gekennzeichnet, daß die Reduktionsreaktion 4 bei 15-20°C durch Wasserstoffreduktion in Gegenwart von Ni, Pt, Pd oder einem Übergangsmetallkatalysator oder durch Hydridreduktion oder mit Wasserstoff in Gegenwart eines Raney-Nickel-Katalysators bei 70-75°C unter einem Druck von 40-55 atm durchgeführt wird.

7. Verfahren gemäß einem der Ansprüche 3-6, dadurch gekennzeichnet, daß die Veresterungsreaktion 5 und die N-Acylierung unter Verwendung einer entsprechenden Carbonsäure, eines Carbonsäurechlorids oder eines Carbonsäureanhydrids bei 20-100°C durchgeführt werden.

8. Verwendung der Verbindungen gemäß Anspruch 1 für Weichmacher und Stabilisierungsmittel bei Polymeren (insbesondere bei PVC-Polymeren), als Schmiermittel, Emulgatoren, Korrosionsschutzmittel und als Metallkomplexierungsmittel.

## Revendications

1. Dérivés N-acyl et O-acyl de la N,N-bis(2,2-diméthyl-3-hydroxypropyl)amine.

2. Composés selon la revendication 1 répondant à la formule : 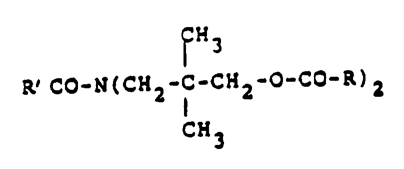 dans laquelle R et R' sont identiques ou différents et sont chacun un groupe alkyle en C₁-C₁₀ ou aryle en C₆-C₁₀.

3. Un procédé pour la production des composés selon la revendication 1 ou la revendication 2, caractérisé en ce que l'on fait réagir ensemble un halogénure ou sulfate d'ammonium, le formaldéhyde et l'isobutyraldéhyde selon l'équation 3. 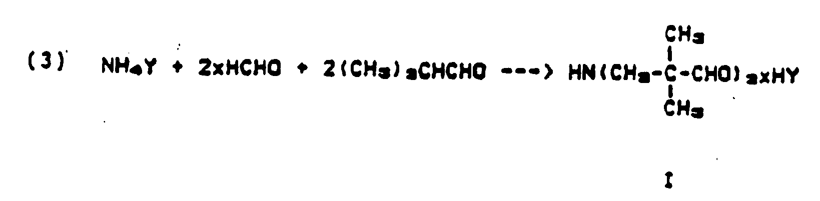 (dans laquelle Y est un halogénure ou un sulfate) dont le produit de condensation est ensuite réduit selon l'équation 4. 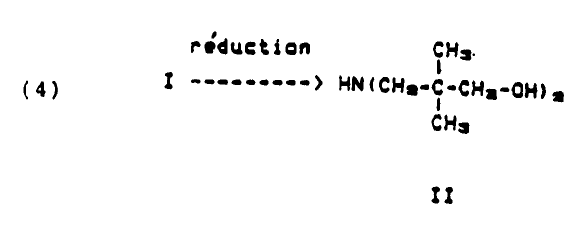 puis, le produit réduit est acylé selon l'équation 5 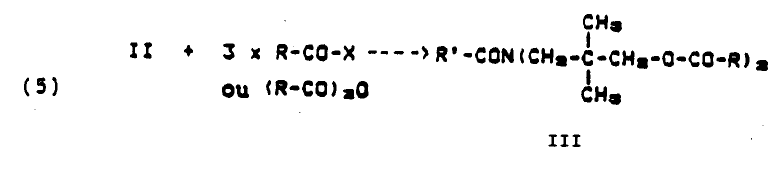 dans laquelle R et R' sont des groupes alkyles identiques ou différents qui peuvent contenir jusqu'à 10 atomes de carbone ; et X est un groupe halogénure ou acyloxy.

4. Un procédé selon la revendication 3, caractérisé en ce que, dans la réaction de condensation de l'équation 3, on utilise comme source de formaldéhyde le paraformaldéhyde, le trioxanne ou une solution aqueuse à 40 % de formaldéhyde.

5. Un procédé selon la revendication 3 ou la revendication 4, caractérisé en ce que la réaction de condensation selon l'équation 3 est effectuée entre 50 et 90°C, à reflux ou sous une légère surpression.

6. Un procédé selon l'une quelconque des revendications 3 à 5, caractérisé en ce que la réaction de réduction 4 est effectuée entre 15 et 20°C par réduction par l'hydrogène en présence d'un catalyseur au Ni, Pt, Pd ou métal de transition, ou par réduction avec un hydrure, ou avec l'hydrogène en présence d'un catalyseur au nickel de Raney, entre 70 et 75°C et à une pression de 40 à 55 atmosphères.

7. Un procédé selon l'une quelconque des revendications 3 à 6, caractérisé en ce que l'on effectue la réaction d'estérification 5 et on effectue la N-acylation en utilisant un acide carboxylique ou un chlorure ou anhydride d'acide carboxylique appropriés, entre 20 et 100°C.

8. L'utilisation des composés selon la revendication 1 comme plastifiants et agents stabilisants dans des polymères (en particulier un polymère de PVC), comme agents lubrifiants, émulsifiants, agents de résistance à la corrosion et agents complexant les métaux.
